# FASCICULE DE BREVET EUROPEEN

(11) **EP 1 448 550 B1**
(45) Date de publication et mention de la délivrance du brevet: **23.02.2011**
(21) Numéro de dépôt: 02803433.8
(22) Date de dépôt: 18.11.2002
(51) Int. Cl.: C07D 401/10, A61K 31/4523, A61K 31/4545, A61P 25/00

(54) **TETRAHYDROPYRIDYL-ALKYL-HETEROCYCLES AZOTES AVEC UNE ACTIVITE DU TNF**
AZOTIERTE TETRAHYDROPYRIDYL-ALKYL-HETEROZYKLEN MIT TNF-WIRKUNG
NITROGENOUS TETRAHYDROPYRIDYL-ALKYL-HETEROCYCLES WITH TNF ACTIVITY

(30) Priorité: 19.11.2001 FR 0114897
(43) Date de publication de la demande: 25.08.2004
(73) Titulaire: Sanofi-Aventis, 75013 Paris (FR)
(72) Inventeur: BARONI, Marco, I-20010 Vanzago-Milano (IT); BOURRIE, Bernard, F-34980 Saint-Gély-du-Fesc (FR); CASELLAS Pierre, F-34090 Montpellier (FR); PULEO, Letizia, I-20000 Milano (IT)
(74) Mandataire: Le Coupanec, Pascale A.M.P.
(86) Numéro de dépôt international: PCT/FR2002/003929
(87) Numéro de publication internationale: WO 2003/044010

(56) Documents cités:
- WO-A-01/29026
- WO-A-98/25903
- WO-A2-99/59526
- JP-A- 49 100 089

## Description

La présente invention concerne de nouveaux tétrahydropyridyl-alkyl-hétérocycles azotés, notamment des hétérocycles monocycliques azotés, les compositions pharmaceutiques les contenant et un procédé pour leur préparation.

WO 98/25903 décrit des 1-phenylalkyl-1,2,3,6-tétrahydropyridines pour le traitement de la maladie d'Alzheimer.

WO 01/29026 décrit des phényl- et pyridyl-tétrahydropyridines possédant une activité inhibitrice du TNF-alpha (de l'anglais Tumour Necrosis Factor).

Bourrié et al. (Proc. Natl. Acad. Sci. 1999, 96(22):12855-12859) ont décrit l'activité d'un composé dénommé SR 57746 (1-(2-napht-2-yl-éthyl)-4-(3-trifluorométhyl)-1,2,3,6-tétrahydropyridine) dans un modèle d'encéphalomyelite autoimmune expérimentale (EAE) et dans la modulation du TNF-alpha.

Le TNF-alpha est une cytokine qui a récemment suscité de l'intérêt en tant que médiateur de l'immunité, de l'inflammation, de la prolifération cellulaire, de la fibrose etc. Ce médiateur est copieusement présent dans le tissu synovial enflammé et exerce un rôle important dans la pathogenèse de l'autoimmunité (Annu. Rep. Med. Chem., 1997, 32:241-250).

Il a été maintenant trouvé que certaines tétrahydropyridines substituées par des dérivés hérétocycliques azotés monocycliques, possèdent une puissante activité vis-à-vis de la modulation du TNF-alpha.

Ainsi, la présente invention concerne, selon un de ses aspects, des tétrahydropyridyl-alkyl-benzodiazines de formule (I) : 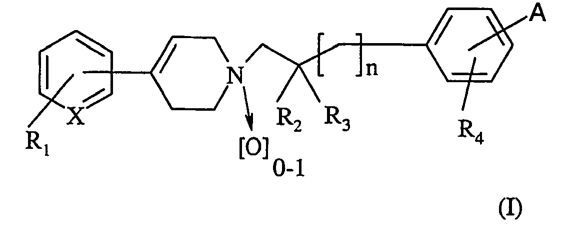 dans laquelle
- X: représente N ou CH ;
- R₁: représente un atome d'hydrogène ou d'halogène ou un groupe CF₃ ;
- R₂ et R₃: représentent indépendamment un atome d'hydrogène ou un groupe (C₁-C₄) alkyle;
- R₄: représente un atome d'hydrogène ou un groupe (C₁-C₄) alkyle;
- n: est 0 ou 1 ;
- A: représente un hétérocycle azoté de formule (a)-(d) : 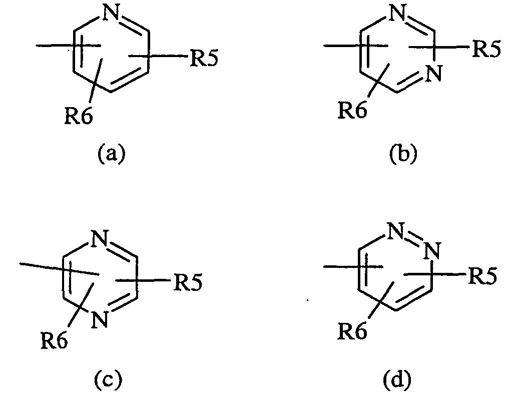
où R₅ et R₆ représentent chacun indépendamment un atome d'hydrogène, un groupe (C₁-C₄) alkyle ou (C₁-C₄) alcoxy ;
ainsi que leurs N-oxydes et leurs sels ou solvates.

Dans la présente description, le terme "(C₁-C₄)alkyle" désigne un radical monovalent d'un hydrocarbure en C₁-C₄ saturé à chaîne droite ou ramifiée et le terme "(C₁-C₄)alcoxy" désigne un radical monovalent d'un hydrocarbure en C₁-C₄ saturé à chaîne droite ou ramifiée lié par l'intermédiaire d'un atome d'oxygène

Dans la présente description, le terme "halogène" désigne un atome choisi parmi le chlore, le brome, le iode et le fluor.

Des composés de formule (I) préférés sont ceux où n est zéro.

D'autres composés préférés sont ceux où R₂ et R₃ sont l'hydrogène.

D'autres composés préférés sont ceux où R₁ est un groupe CF₃.

D'autres composés préférés sont ceux où X est CH et R₁ est dans la position 3 du benzène.

D'autres composés préférés sont ceux où X est CH et R₁ est un groupe CF₃.

D'autres composés préférés sont ceux où X est N et la pyridine ainsi formée est substituée dans les positions 2,6.

D'autres composés préférés sont ceux où le noyau phényle est substitué par l'hétérocycle azoté A dans la position 3.

Les hétérocycles azotés de formule (a)-(d) représentent respectivement une pyridine, une pyrimidine, une pyrazine et une pyridazine. Ces hétérocycles pourront, selon la présente invention, être rattachés au reste de la molécule de formule (I) par l'un quelconque des atomes de carbone des positions disponibles.

Selon la présente invention, les composés de formule (I) peuvent exister comme dérivés N-oxydes. Comme indiqué dans la formule ci-dessus, les composés de formule (I) peuvent notamment porter le groupe N-oxyde sur la tétrahydropyridine; alternativement des groupes N-oxyde peuvent être présents sur les azotes des groupes (a)-(d) et, éventuellement, tous les azotes des composés de formule (I) pourront être simultanément oxydés.

Les sels des composés de formule (I) selon la présente invention comprennent aussi bien les sels d'addition avec des acides minéraux ou organiques pharmaceutiquement acceptables tels que le chlorhydrate, le bromhydrate, le sulfate, l'hydrogénosulfate, le dihydrogénophosphate, le citrate, le maléate, le tartrate, le fumarate, le gluconate, le méthanesulfonate, le 2-naphtalènesulfonate, etc., que les sels d'addition qui permettent une séparation ou une cristallisation convenable des composés de formule (I), tels que le picrate, l'oxalate ou les sels d'addition avec des acides optiquement actifs, par exemple les acides camphosulfoniques et les acides mandéliques ou mandéliques substitués.

Les stéréoisomères optiquement purs, ainsi que les mélanges d'isomères des composés de formule (I), dus au carbone asymétrique, quand l'un de R₂ et R₃ sont différents l'un de l'autre, dans une proportion quelconque, font partie de la présente invention.

Les composés de formule (I) peuvent être préparés par une réaction de condensation à partir d'un composé de formule (II) : 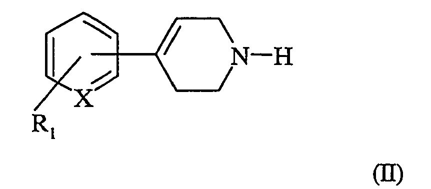 dans laquelle X et R₁ sont tels que définis ci-dessus, avec un composé de formule (III) : 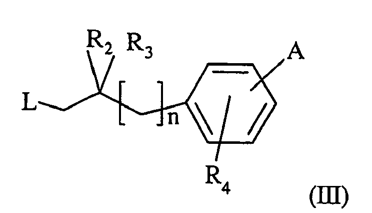 dans laquelle R₂, R₃, R₄, n et A sont tels que définis précédemment et L est un groupe partant, isolement du composé de formule (I) et transformation éventuelle en un de ses sels ou solvates ou dans ses dérivés N-oxydes.

La réaction de condensation est normalement conduite en mélangeant les composés de départ (II) et (III) dans un solvant organique inerte, selon les méthodes conventionnelles.

Par "solvant organique inerte" on entend un solvant qui n'interfère pas avec la réaction. De tels solvant sont par exemple les alcools tel que le méthanol, l'éthanol, le propan-2-ol ou le butanol.

Comme groupe partant L on peut par exemple utiliser un halogène tel qu'un atome de chlore ou de brome, ou bien un groupe méthylsulfonyloxy (CH₃SO₂-O-).

La réaction est conduite à une température comprise entre -10°C et la température de reflux du mélange réactionnel, la température de reflux étant préférée.

La réaction peut être convenablement conduite en présence d'un accepteur de protons, par exemple d'un carbonate alcalin ou d'une amine tertiaire telle que la triéthylamine.

La réaction est généralement terminée après quelques heures, normalement de 1 à 6 heures suffisent pour compléter la condensation.

Le composé voulu est isolé selon les techniques conventionnelles sous forme de base libre ou d'un de ses sels. La base libre peut être transformée dans un de ses sels par simple salification dans un solvant organique tel qu'un alcool, de préférence l'éthanol ou l'isopropanol, un éther comme le 1,2-diméthoxyéthane, l'acétate d'éthyle, l'acétone ou un hydrocarbure comme l'hexane.

Le composé de formule (I) obtenu est isolé selon les techniques usuelles et éventuellement transformé en un de ses sels ou solvates ou dans ses dérivés N-oxyde.

Les composés de départ de formule (II) sont connus ou bien ils peuvent être préparés de façon analogue aux composés connus.

Les composés de départ de formule (III) sont nouveaux et constituent un objet ultérieur de la présente invention. Ces composés peuvent être préparés à partir des acides ou esters correspondants, par réduction du groupe carboxylique en groupe alcool et substitution de l'OH par le groupe L souhaité selon les méthodes conventionnelles. Alternativement, ces composés sont préparés par condensation de Stille ou de Suzuki des deux noyaux phényle et hétérocycle, convenablement substitués; selon ces condensations, un halo-phényle convenable est mis à réagir avec respectivement des dérivés des tri-alkylstannanes ou des diboranes du noyau hétérocycle ou, inversément, un halo-hétèrocycle approprié est mis à réagir avec respectivement des dérivés des tri-alkylstannanes ou des diboranes du noyau phényle. Après la condensation, les noyaux sont éventuellement fonctionalisés par transformation des groupes présents, selon les méthodes bien connues. Des exemples de préparation de trialkylstannane-hétérocycliques azotés sont rapportés dans Bioroganic and Médicinal Chemistry, 9/2001, 2683-2691. D'autres exemples des réactions ci-dessus sont consignés dans la partie expérimentale.

Alternativement, les composés de formule (I) peuvent être préparés par un procédé qui prévoit :
(a) de faire réagir le composé de formule (VI): 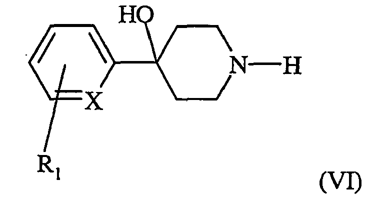 dans laquelle X et R₁ sont définis comme précédemment, avec un dérivé fonctionnel de l'acide de formule (VII) 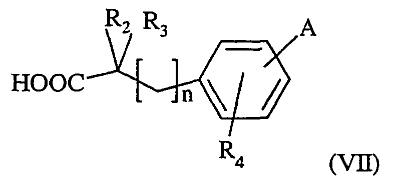 dans laquelle R₂, R₃, R₄, n et A sont tels que définis ci-dessus,
(b) de réduire le groupe carbonyle du composé de formule (VIII) ainsi obtenu: 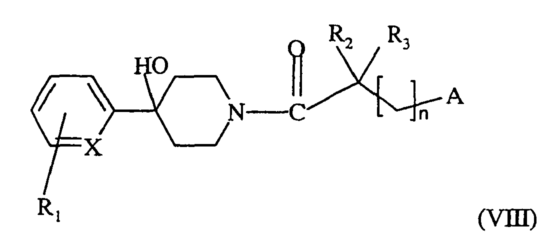
(c) de déshydrater le pipéridinol intermédiaire de formule (IX) ainsi obtenu: 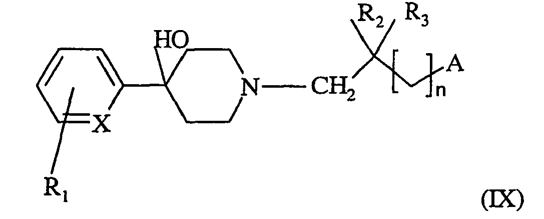
(d) d'isoler le composé de formule (I) ainsi obtenu et, éventuellement le transformer en l'un de ses sels ou solvates ou dans ses dérivés N-oxyde.

La réaction de l'étape (a) peut être convenablement conduite dans un solvant organique à une température comprise entre -10°C et la température de reflux du mélange réactionnel.

Il peut être préférable de réaliser la réaction à froid lorsqu'elle est exothermique, comme dans le cas où on utilise le chlorure en tant que dérivé fonctionnel de l'acide de formule (VII).

Ces composés peuvent être préparés à partir des produits obtenus par les condensations de Stille ou Suzuki ci-dessus mentionnées, notamment par transformation des substituants présents sur le phényle en les groupes acides souhaités.

Comme dérivé fonctionnel approprié de l'acide de formule (VII), on peut utiliser l'acide libre, éventuellement activé (par exemple avec le BOP = tri(diméthylamino)benzotriazol-1-yloxyphosphonium hexafluorophosphate), un anhydride, un anhydride mixte, un ester actif ou un halogénure d'acide, le bromure de préférence. Parmi les esters actifs, l'ester de p-nitrophényle est particulièrement préféré, mais les esters de méthoxyphényle, de trityle, de benzhydryle et similaires sont également convenables.

Comme solvant de réaction, on utilise de préférence un solvant halogéné tel que le chlorure de méthylène, le dichloroéthane, le 1,1,1-trichloroéthane, le chloroforme et similaires, mais aussi d'autres solvants organiques compatibles avec les réactifs employés, par exemple le dioxane, le tétrahydrofurane ou un hydrocarbure tel que l'hexane, peuvent être également employés.

La réaction peut être convenablement conduite en présence d'un accepteur de protons, par exemple d'un carbonate alcalin ou d'une amine tertiaire telle que la triéthylamine.

La réduction de l'étape (b) peut être convenablement réalisée par des agents de réduction appropriés tels que les complexes du borane, par exemple le diméthylsulfure de borane ([CH₃]₂S-BH₃), les hydrures d'aluminium ou un hydrure complexe de lithium et d'aluminium dans un solvant organique inerte à une température comprise entre 0°C et la température de reflux du mélange réactionnel, selon les techniques usuelles.

Par "solvant organique inerte" on entend un solvant qui n'interfère pas avec la réaction. De tels solvants sont par exemple les éthers, tel que l'éther diéthylique, le tétrahydrofurane (THF), le dioxane ou le 1,2-diméthoxyéthane.

Alternativement, les composés de formule (I) peuvent également être préparés par une réaction de condensation/réduction à partir d'un composé de formule (II) : 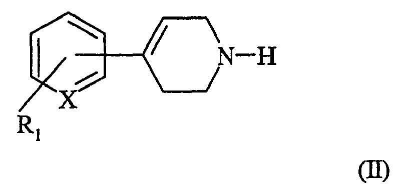 dans laquelle X et R₁ sont tels que définis ci-dessus, avec un aldéhyde de formule (X) : 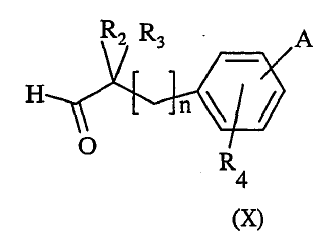 dans laquelle R₂, R₃, R₄, n et A sont tels que définis précédemment, isolement du composé de formule (I) et transformation éventuelle en un de ses sels ou solvates ou dans ses dérivés N-oxydes.

La réaction de condensation/réduction est conduite en mélangeant les composés de départ (II) et (X) dans un solvant organique tel qu'un alcool tel que par exemple le méthanol, en milieu acide, en présence d'un agent de réduction tel que le cyano-borohydrure de sodium, selon les méthodes conventionnelles.

Les composés de formule (I) portant un groupe N-oxyde sur les atomes d'azote des groupes (a)-(d) peuvent être préparés à partir des dérivés N-oxyde des composés de formule (III) ou (X).

Les composés de formule (I) portant un groupe N-oxyde sur l'atome d'azote de la tétrahydropyridine ou de la pyridine liée à la tétrahydropyridine lorsque X est N, peuvent être préparés par oxydation des composés de formule (I) correspondant. Dans ce cas, le composé de formule (I) tel qu'obtenu par exemple par la synthèse ci-dessus, est soumis à une réaction d'oxydation selon les méthodes conventionnelles, par exemple à une réaction avec de l'acide *m-*chloro-perbenzoïque dans un solvant convenable et isolé selon les techniques usuelles bien connues à l'homme du métier.

Les composés de l'invention possèdent des propriétés intéressantes vis-à-vis de l'inhibition du TNF-α.

Ces propriétés ont été mises en évidence à l'aide d'un test visant à mesurer l'effet de molécules sur la synthèse du TNF-α induite chez la souris Balb/c par du lipopolysaccharide (LPS) d'Escherichia Coli (055 :B5, Sigma, St Louis, Mo).

Les produits à tester sont administrés par voie orale à des groupes de 5 souris Balb/c femelles (Charles River, France) âgées de 7 à 8 semaines. Une heure après, le LPS est administré par voie intravéneuse (10µg/souris). Le sang de chaque animal est prélevé 1,5 heure après l'administration du LPS. Les échantillons sont centrifugés, le plasma est récupéré et congelé à -80°C. Le TNF-α est mesuré à l'aide de kits commerciaux (R et D, Abingdon, UK).

Dans ce test, des composés représentatifs de l'invention se sont montrés très actifs, en inhibant la synthèse du TNF-α même à doses très faibles.

Grâce à cette activité et à leur faible toxicité, les composés de formule (I) et ses sels ou solvates peuvent bien être utilisés dans le traitement des maladies liées à des troubles immunitaires et inflammatoires ou comme analgésiques. Notamment les composés de formule (I) peuvent être utilisés pour traiter l'athérosclérose, les maladies autoimmunes, les maladies qui entraînent la démyélinisation des neurones (telles que la sclérose en plaques), l'asthme, l'arthrite rhumatoïde, les maladies fibrotiques, la fibrose idiopathique pulmonaire, la fibrose cystique, la glumérulonéphrite, la spondylite rhumatoïde, l'ostéoarthrite, la goutte, la résorption osseuse et cartilagineuse, l'ostéoporose, la maladie de Paget, le myélome multiple, l'uvéorétinite, les chocs septiques, la septicémie, les chocs endotoxiniques, la réaction du greffon contre l'hôte, le rejet des greffes, le syndrome de détresse respiratoire de l'adulte, la silicose, l'asbestose, la sarcoïdose pulmonaire, la maladie de Crohn, la colite ulcérative, la sclérose latérale amyotrophique, la maladie d'Alzheimer, la maladie de Parkinson, le lupus érythémateux disséminé, les chocs hémodynamiques, les pathologies ischémiques (infarctus myocardique, ischémie myocardique, vasospasme coronarien, angine de poitrine, insuffisance cardiaque, attaque cardiaque), les atteintes post ischémiques de reperfusion, la malaria, les infections mycobactériennes, la méningite, la lèpre, les infections virales (HIV, cytomegalovirus, virus de l'herpès), les infections opportunistes liées au Sida, la tuberculose, le psoriasis, la dermatose atopique et de contact, le diabète, la cachexie, le cancer, les dommages liés aux radiations.

Les composés de formule (I) et leurs sels et solvates pharmaceutiquement acceptables sont de préférence administrés par voie orale.

Dans les compositions pharmaceutiques de la présente invention par voie orale, le principe actif peut être administré sous formes unitaires d'administration, en mélange avec des supports pharmaceutiques classiques, aux animaux et aux êtres humains pour le traitement des affections susmentionnées. Les formes unitaires d'administration appropriées comprennent par exemple les comprimés éventuellement sécables, les gélules, les poudres, les granules et les solutions ou suspensions orales.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Le principe actif peut être formulé également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters.

La quantité de principe actif à administrer dépend comme toujours du degré d'avancement de la maladie ainsi que de l'âge et du poids du patient. Néanmoins, les doses unitaires comprennent généralement de 0,001 à 100 mg, mieux de 0,01 à 50 mg, de préférence de 0,1 à 20 mg de principe actif, avantageusement de 0,5 à 10 mg.

Selon un autre de ses aspects, la présente invention concerne une association comprenant un composé de formule (I) ou l'un de ses sels ou solvates pharmaceutiquement acceptables, et au moins un composé choisi parmi les agents immunosuppresseurs, tel que l'interféron bêta-1b; l'hormone adrénocorticotrope; les glucocorticoïdes tels que la prednisone ou la méthylprednisolone; les inhibiteurs de finterleukine-1.

Plus particulièrement, l'invention concerne une association comprenant un composé de formule (I), ou l'un de ses sels ou solvates pharmaceutiquement acceptables et au moins un composé choisi parmi le roquinimex (1,2-dihydro-4-hydroxy-N,1-diméthyl-2-oxo-3-quinolinecarboxanilide), le myloran (produit de la société Autoimmune contenant de la myéline bovine), l'antegren (anticorps humain monoclonal des sociétés Elan/Athena Neurosciences) l'interféron bêta-lb recombinant.

D'autres associations possibles sont celles constituées par un composé de formule (I) ou l'un de ses sels ou solvates pharmaceutiquement acceptables et un bloqueur des canaux potassiques, tel que par exemple la fampridine (4-aminopyridine).

Selon un autre de ses aspects, l'invention concerne une méthode de traitement des maladies liées à des troubles immunitaires et inflammatoires ainsi que dans le traitement de la douleur, notamment l'athérosclérose, les maladies autoimmunes, les maladies qui entraînent la démyélinisation des neurones (telles que la sclérose en plaques), l'asthme, l'arthrite rhumatoïde, les maladies fibrotiques, la fibrose idiopathique pulmonaire, la fibrose cystique, la glumérulonéphrite, la spondylite rhumatoïde, l'ostéoarthrite, la goutte, la résorption osseuse et cartilagineuse, l'ostéoporose, la maladie de Paget, le myélome multiple, l'uvéorétinite, les chocs septiques, la septicémie, les chocs endotoxiniques, la réaction du greffon contre l'hôte, le rejet des greffes, le syndrome de détresse respiratoire de l'adulte, la silicose, l'asbestose, la sarcoïdose pulmonaire, la maladie de Crohn, la colite ulcérative, la sclérose latérale amyotrophique, la maladie d'Alzheimer, la maladie de Parkinson, le lupus érythémateux disséminé, les chocs hémodynamiques, les pathologies ischémiques (infarctus myocardique, ischémie myocardique, vasospasme coronarien, angine de poitrine, insuffisance cardiaque, l'attaque cardiaque), les atteintes post ischémiques de reperfusion, la malaria, les infections mycobactériennes, la méningite, la lèpre, les infections virales (HIV, cytomegalovirus, virus de l'herpès), les infections opportunistes liées au Sida, la tuberculose, le psoriasis, la dermatose atopique et de contact, le diabète, la cachexie, le cancer, les dommages liés aux radiations, comprenant l'administration d'un composé de formule (I) ou de l'un de ses sels ou solvates pharmaceutiquement acceptables, seul ou en association avec d'autres principes actifs.

Selon un aspect ultérieur, l'invention concerne un médicament contenant, en tant que princpe actif, au moins un composé de formule (I) ou l'un de ses sels ou solvates pharmaceutiquement acceptables.

Les exemples qui suivent illustrent l'invention.

### PREPARATION 1

### 3-[4-(2-Méthylsulfonyloxy-éthyl)-phényl]-pyridine.

### (i) 3-[4-(2-hydroxyéthyl)-phényl]-pyridine

On mélange 1 g (6,8 mmole) de 3-pyridyl-diéthylborane, 0,87 ml (10,2 mmole) de 2-(4-bromophényl)-éthanol, 1,53 g (27 mmole) de KOH en poudre, 1,1 g (3,4 mmole) de bromure de tétrabutylammonium et 0,39 g (0,34 mmole) de (triphénylphosphine)Pd-tétrakis dans 25 ml de THF anhydre. On chauffe au reflux sous courant d'argon pendant 4 heures. On verse le mélange dans de l'eau, on extrait à l'acétate d'éthyle, on sèche la phase organique et on évapore le solvant. On purifie le résidu par chromatographie sur colonne de gel de silice en éluant par un mélange cyclohexane/acétate d'éthyle=1/1 en obtenant ainsi le produit du titre.

### (ii) 3-[4-(2-méthylsulfonyloxy-éthyl)-phényl]-pyridine

On dissout 1 g (4,6 mmole) du produit de l'étape précédente dans 6,9 ml de chlorure de méthylène, et on refroidit à 0°C. On ajoute 0,64 ml de triéthylamine et après, goutte à goutte, une solution de 0,36 ml de (4,6 mmole) methansulfonylchlorure dans 0,46 ml de chlorure de méthylène. On agite à 0°C pendant 30 minutes et ensuite à la température ambiante pendant 4 heures. On verse le mélange dans de l'eau, on extrait à l'acétate d'éthyle, on sèche la phase organique et on évapore le solvant. On purifie le résidu par chromatographie sur colonne de gel de silice en éluant par un mélange cyclohexane/acétate d'éthyle=2/8, en obtenant ainsi le produit du titre.
P.f. 66-68°C

### PREPARATION 2

### 2-[4-(2-Méthylsulfonyloxy-éthyl)-phényl]-pyridine.

### (i) 2-[4-(2-hydroxyéthyl)-phényl]-pyridine

On refroidit à -78°C sous courant d'argon, 1 ml (10,5 mmole) de 2-bromopyridine dans 80 ml de THF anhydre et on y ajoute 9,4 ml (15 mmole) de n-BuLi, 1,6 N dans l'hexane et on agite pendant 30 minutes. On ajoute donc 3,2 ml (12 mmole) de chlorure de tributylétain et on agite toujours à -78°C pendant 2 heures et après à -20°C pendant 3 heures. On verse dans une solution acqueuse de NH₄Cl et on extrait à l'acétate d'éthyle. On sèche la phase organique et on évapore le solvant. On purifie le résidu par chromatographie sur colonne de gel de silice en éluant par un mélange cyclohexane/acétate d'éthyle=9/1 en obtenant ainsi 2,5 g de 2-tributylétain-pyridine sous forme de huile jaune. On dissout 0,31 g du produit ainsi obtenu dans 5 ml de toluène et on y ajoute 0,13 ml (0,92 mmole) de 2-(4-bromophényl)-éthanol et 49 mg (0,042 mmole) de (triphénylphosphine)Pd-tétrakis. On chauffe au reflux sous courant d'argon pendant 6 heures. On verse le mélange dans de l'eau, on extrait à l'acétate d'éthyle, on sèche la phase organique et on évapore le solvant. On purifie le résidu par chromatographie sur colonne de gel de silice en éluant par un mélange cyclohexane/acétate d'éthyle=1/1 et après 4/6, en obtenant ainsi le produit du titre. (ii) 2-[4-(2-méthylsulfonyloxy-éthyl)-phényl]-pyridine En opérant comme décrit dans la Préparation 1 (ii) mais en utilisant le produit de l'étape précédente au lieu du produit de la Préparation 1 (i), on obtient le composé du titre.

### PREPARATION 3

### Acide (4-(4-pyridyl)-phényl)-acétique.

### (i) 4-(4-pyridyl)-acétophénone

On mélange 0,85 g (5,6 mmole) de 4-bromo-pyridine, 1 g (6,1 mmole) d'acide 4-acétylphényl-boronique, 1,26 g (12 mmole) de carbonate de sodium et 260 mg de (triphénylphosphine)Pd-tétrakis dans 25 ml de THF et 6 ml d'eau. On chauffe à 90°C sous courant d'azote pendant 2 heures. On verse le mélange dans de l'eau, on extrait à l'éther diéthylique, on sèche la phase organique et on évapore le solvant. On purifie le résidu par chromatographie sur colonne de gel de silice en éluant par un mélange cyclohexane/acétate d'éthyle=3/7 en obtenant ainsi le produit du titre sous forme de solide blanc.
P.f. 88-90°C

### (ii) acide (4-(4-pyridyl)-phényl)-acétique

On mélange 1,5 g (7,6 mmole) du produit l'étape précédente, 0,29 g de soufre, 3,6 ml de morpholine et une quantité catalytique d'acide p-toluènsulfonique monohydraté et on chauffe sous courant d'azote à 130°C. Après 6 heures, on refroidit, on ajoute 7 ml d'éthanol absolu et on agite à la température ambiante pendant une nuit. On dissout 1,5 g du thioamide ainsi formé dans une solution de 12,3 ml d'éthanol, 56,2 ml d'eau et 0,6 g de NaOH et on chauffe au reflux pendant 3 heures. On acidifie ensuite avec une solution diluée d'acide chlorhydrique et on évapore les solvants. On obtient ainsi le composé du titre.

### PREPARATION 4

### Acide (4-(6-méthyl-pyrid-3-yl)-phényl)-acétique.

### (i) 4-(6-méthyl-pyrid-3-yl)-acétophénone

On mélange 0,25 g (1,1 mole) de 3-trifluorométhylsulfonate-6-méthyl-pyridine, 0,18 g (1 mmole) d'acide 4-acétylphényl-boronique, 1,3 ml d'une solution 2M de carbonate de sodium et 38 mg de (triphénylphosphine)Pd-tétrakis et 85 mg (2 mmole) de LiCl dans 9 ml de 1,2-diméthoxyéthane anhydre. On chauffe à 75°C pendant 3 heures. On verse le mélange dans de l'eau, on extrait à l'éther diéthylique, on sèche la phase organique et on évapore le solvant.On traite le résidu dans de l'hexane et on obtient ainsi le produit du titre sous forme de solide blanc.
P.f. 94-96°C

### (ii) acide (4-(6-méthyl-pyrid-3-yl)-phényl)-acétique

En opérant comme décrit dans la Préparation 3 (ii) mais en utilisant le produit de l'étape précédente au lieu du produit de la Préparation 3 (i) on obtient le composé du titre.

### PREPARATION 5

### Bromhydrate de 3-[3-(2-bromoéthyl)-phényl]-pyridine.

### (i) 3-[3-(2-hydroxyéthyl)-phényl]-pyridine

En opérant comme décrit dans la Préparation 1 mais en utilisant le 2-(3-bromophényl)-éthanol au lieu du 2-(4-bromophényl)-éthanol, on obtient le composé du titre.

### (ii) bromhydrate de 3-[3-(2-bromoéthyl)-phényl]-pyridine

On dissout le produit de l'étape précédente dans 6 ml de acide bromhydrique à 48% et on chauffe à 125°C pandant 6 heures. On dilue avec 2 ml d'eau et on filtre le produit précipité. On obtient ainsi le produit du titre.

### PREPARATION 6

### Acide (4-(5-pyrimidinyl)-phényl)-acétique.

### (i) 4-(5-pyrimidinyl)-acétophénone

En opérant comme décrit dans la Préparation 4 mais en utilisant le la 5-bromopyrimidine au lieu de la 3-trifluorométhansulfonate-6-méthyl-pyridine, on obtient lecomposé du titre
P.f. 134-136°C.

### (ii) acide (4-(5-pyrimidinyl)-phényl)-acétique

En opérant comme décrit dans la Préparation 3 (ii) mais en utilisant le produit de l'étape précédente au lieu du produit de la Préparation 3 (i) on obtient le composé du titre.

### PREPARATION 7

### 4-[3-(2-Bromoéthyl)-phényl]-pyridine.

En opérant comme décrit dans la Préparation 5 mais en utilisant la 4-(4,4,5,5-tétraméthyl[1,3,2]dioxaboran-2-yl)-pyridine au lieu du 3-pyridyl-diéthylborane, on obtient le composé du titre sous forme d'huile jaune.

### PREPARATION 8

### -[3-(2-Chloroéthyl)-phényl]-pyridine.

### (i) 2-[3-(2-hydroxyéthyl)-phényl]-pyridine

En opérant comme décrit dans la Préparation 2(i) mais en utilisant le 2-(3-bromophényl)-éthanol au lieu du le 2-(4-bromophényl)-éthanol, on obtient le composé du titre sous forme d'huile jaune.

### (ii) 2-[3-(2-chloroéthyl)-phényl]-pyridine

On réfroidit à 0°C le produit de l'étape précédente dans 17 ml de dichlorométhane et on y ajoute 11 ml de SOCl₂. On agite pendant une nuit à la température ambiante, on verse dans un mélange d'eau/glace, on porte à pH neutre par addition de bicarbonate de sodium, on extrait avec du dichlorométhane, on sèche et on évapore le solvant. On obtient le composé du titre sous forme d'huile jaune.

### PREPARATION 9

### 4-[3-(2-Chloroéthyl)- phényl]-pyridine.

### (i) 4-[3-(2-hydroxyéthyl)-phényl]-pyridine

En opérant comme décrit dans la Préparation 5(i) mais en utilisant la 4-(4,4,5,5-tétraméthyl[1,3,2]dioxaboran-2-yl)-pyridine au lieu du 3-pyridyl-diéthylborane, on obtient le composé du titre sous forme d'huile jaune.

### (ii) 4-[3-(2-chloroéthyl)-phényl]-pyridine

En opérant comme décrit dans la Préparation 8(ii) mais en utilisant le produit de l'étape précédente, on obtient le composé du titre sous forme d'huile jaune.

### PREPARATION 10

### 3-[3-(2-Chloroéthyl)-4-méthyl-phényl]-pyridine.

### (i) 4-[3-(2-hydroxyéthyl)-4-méthyl-phényl]-pyridine

En opérant comme décrit dans la Préparation 1(i) mais en utilisant le 2-(3-bromo-4-méthyl-phényl)-éthanol au lieu du 2-(4-bromophényl)-éthanol on obtient le composé du titre.

### (ii) 4-[3-(2-chloroéthyl)-4-méthyl-phényl]-pyridine

En opérant comme décrit dans la Préparation 8(ii) mais en utilisant le produit de l'étape précédente, on obtient le composé du titre.

### PREPARATION 11

### 3-(4-pyridyl-1-oxyde)-phényl-acétaldéhyde.

On refroidit à 0°C 2,5 g (0,015 mole) de 4-(3-hydroxy-phényl)-pyridine et 40 ml de pyridine et 2,3 g de 4-diméthylamino-pyridine (DMAP). On y ajoute goutte à goutte 3,2 ml d'anhydride triflique. On agite pendant 1 heure à 0°C et après une nuit à la température ambiante. On verse dans un mélange eau/glace, on extrait à l'acétate d'éthyle, on sèche la phase organique et on évapore le solvant sous pression réduite. On purifie le brut par chromatographie sur colonne de gel de silice en éluant par un mélange cyclohexane/acétate d'éthyle = 8/2. On obtient la 4-(3-hydroxy-phényl)-pyridine trifluorométhanesulfonate sous forme d'huile. On mélange 2,26 g de ce produit avec 112 ml de diméthylformamide anhydre, 38 mg de acétate de palladium, 2,5 ml de triéthylamine anhydre, 2 g de N,N-diméthyléthanolamine vinyléther. On chauffe à 80°C pendant 48 heures. On évapore le solvant sous pression réduite. On purifie le résidu par flash-chromatographie sur colonne de gel de silice en éluant par de l'acétate d'éthyle pour éliminer le premier produit après par un mélange acétate d'éthyle/méthanol = 1/1. On obtient la 2-[2-[3-(4-pyridyl)-phényl)éthenyl)oxy)]-N,N-diméthyl-1-éthanamine. On traite 0,53 g de ce produit dissous dans 35 ml de méthanol avec 20 ml d'eau et 5 ml d'acide sulfurique à 96%. On chauffe pendant 6 heures à 60°C, on verse dans de la glace, on y ajoute une solution aqueuse saturée en NaHCO₃ et on extrait à l'acétate d'éthyle. On sèche la phase organique et on évapore le solvant sous pression réduite. On obtient le 3-(4-pyridyl)-phényl-acétaldéhyde diméthylacétal. On prépare son dérivé N-oxyde par réaction avec de l'acide *m-*chloroperbenzoïque dans du chlorure de méthylène et on libère ensuite l'aldéhyde par réaction avec un mélange d'acide trifluoroacétique, eau et chlorure de méthylène. On obtient ainsi de composé du titre.

### EXEMPLE 1

### 1-(2-(4-(3-Pyridyl)-phényl)-éthyl)-4-(3-trifluoro-méthyl-phényl)-1,2,3,6-tétrahydro-pyridine et son dichlorhydrate.

On dissout 0,85 g (2,9 mmole) du produit de la Préparation 1 dans 14 ml de l'isopropanol et on y ajoute 1,21 ml de triéthylamine et 700 mg (2,9 mmole) de 4-(3-trifluoro-méthyl-phényl)-1,2,3,6-tétrahydropyridine. On chauffe au reflux pendant 4 heures, on verse dans l'eau et on extrait à l'acétate d'éthyle. On sèche la phase organique, on évapore le solvant sous pression réduite et on obtient le produit brut qu'on purifie par chromatographie sur colonne de gel de silice en éluant par de l'acétate d'éthyle. On obtient ainsi le produit du titre. On prépare son sel dichlorhydrate par réaction avec de l'acide d'une solution d'isopropanol saturé en acide chlorhydrique.
P.f. 235-237°C (dichlorhydrate)

### EXEMPLE 2

### 1-[2-(4-(2-Pyridyl)-phényl)-éthyl]-4-(3-trifluoro-méthyl-phényl)-1,2,3,6-tétrahydro-pyridine et son dichlorhydrate.

En opérant comme décrit dans l'Exemple 1 mais en utilisant le produit de la Préparation 2, on obtient le composé du titre. On prépare le chlorhydrate à l'aide d'une solution d'isopropanol saturé en acide chlorhydrique.
P.f. 253-256°C (chlorhydrate).

### EXEMPLE 3

### 1-[2-(4-(4-Pyridyl)-phényl)-éthyl]-4-(3-trifluoro-méthyl-phényl)-1,2,3,6-tétrahydro-pyridine et son dichlorhydrate.

On dissout 2,4 g du produit de la Préparation 3 dans 20 ml de chlorure de méthylène et on y ajoute 1,23 g (5 mmole) de 4-hydroxy-4-(3-trifluorométhyl-phényl)pipéridine, 2,2 g (5 mmole) de B.O.P., 2,1 ml de triéthylamine et on agite pendant une nuit, à la température ambiante. On ajoute un mélange d'acétate d'éthyle et d'eau, on sépare les deux phases, on sèche la phase organique sur du sulfate de sodium et on évapore le solvant. On purifie le brut par chromatographie sur colonne de gel de silice en éluant par un mélange acétate d'éthyle/méthanol= 9/1. On verse 1,1 g du produit ainsi obtenu, dissout dans 32 ml de THF anhydre, dans une suspension de 0,19 g (5 mmole) de LiAlH₄ dans 4,5 ml de THF anhydre à 0°C sous courant d'azote. On agite à la température ambiante pendant 5 heures, on verse dans un mélange eau/glace et on extrait à l'acétate d'éthyle. On sépare les deux phases, on sèche la phase organique sur du sulfate de sodium et on évapore le solvant. On dissout 0,65 g du produit ainsi réduit dans 3,9 ml d'acide acétique, on y ajoute 0,78 ml d'acide sulfurique à 96% et on chauffe à 80°C pendant 2 heures. On verse dans de un mélange NaOH/glace et on extrait à l'acétate d'éthyle. On lave à l'eau, on sèche et on évapore sous pression réduite. On purifie le brut par chromatographie sur colonne de gel de silice en éluant par un mélange acétate d'éthyle/méthanol= 95/5. On obtient ainsi le composé du titre. On prépare le sel dichlorhydrate à l'aide d'une solution d'isopropanol saturé en acide chlorhydrique.
P.f. 179-181°C (dichlorhydrate).

### EXEMPLE 4

### 1-[2-(4-(6-Méthyl-pyrid-3-yl)-phényl)-éthyl]-4-(3-trifluoro-méthyl-phényl)-1,2,3,6-tétrahydro-pyridine et son dichlorhydrate.

En opérant comme décrit dans l'Exemple 3 mais en utilisant le produit de la Préparation 4, on obtient le composé du titre.
P.f. 174-175°C (dichlorhydrate).

### EXEMPLE 5

### 1-[2-(3-(3-Pyridyl)-phényl)-éthyl]-4-(3-trifluoro-méthyl-phényl)-1,2,3,6-tétrahydro-pyridine et son dioxalate.

En opérant comme décrit dans l'Exemple 1 mais en utilisant le produit de la Préparation 5 (base libre) du butanol au lieu du propan-2-ol et du carbonate de potassium au lieu de la triéthylamine, on obtient le composé du titre. On prépare son sel oxalate par traitement de la base avec de l'acide oxalique dans de l'acétone.
P.f. 152-157°C (dioxalate).

### EXEMPLE 6

### 1-[2-(4-(5-pyrimidinyl)-phényl)-éthyl]-4-(3-trifluoro-méthyl-phényl)-1,2,3,6-tétrahydro-pyridine et son chlorhydrate.

En opérant comme décrit dans l'Exemple 3 mais en utilisant le produit de la Préparation 6, on obtient le composé du titre.
P.f. 196-198°C (chlorhydrate).

### EXEMPLE 7

### 1-[2-(3-(4-Pyridyl)-phényl)-éthyl]-4-(3-trifluoro-méthyl-phényl)-1,2,3,6-tétrahydro-pyridine et son sel dioxalate.

En opérant comme décrit dans l'Exemple 1 mais en utilisant le produit de la Préparation 7, on obtient le composé du titre sous forme d'huile. On prépare son sel oxalate par traitement de la base avec de l'acide oxalique dans de l'acétone.
P.f. 140-144°C (dioxalate).

### EXEMPLES 8 à 16

En suivant le schéma de réaction de l'Exemple 1 mais en utilisant des produits de départ appropriés, on obtient les composés rapportés dans le Tableau ci-dessous:

| | | |
|---|---|---|
| EX. 8 ⁽¹⁾ | 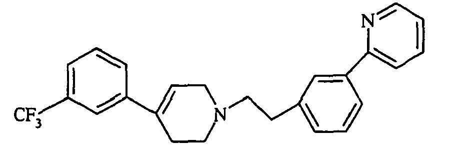 | Sel oxalate P.f. 182-186°C |
| EX. 9 ⁽¹⁾ | 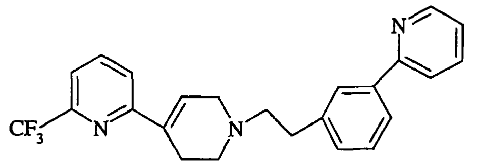 | Sel oxalate P.f. 126-129°C |
| EX. 10 ⁽¹⁾ | 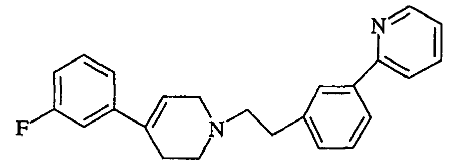 | Sel oxalate P.f. 175-177°C |
| EX. 11 ⁽²⁾ | 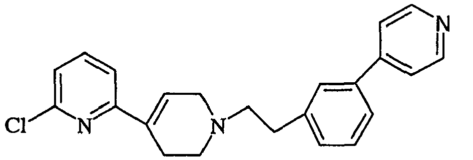 | Sel oxalate P.f. 183-186°C |
| EX. 12 ⁽³⁾ | 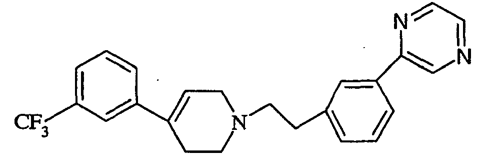 | Sel oxalate P.f. 205-207°C |
| EX. 13 ⁽³⁾ | 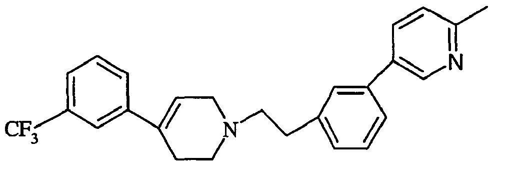 | Sel oxalate P.f. 170-173°C |
| EX. 14 ⁽³⁾ | 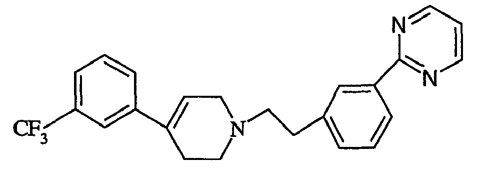 | Sel oxalate P.f. 199-201°C |
| EX. 15 ⁽³⁾ | 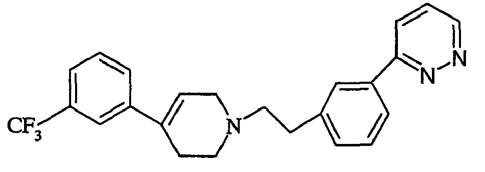 | Masse: M⁺ 409 TLC: R.f. 0,35 (AcEt/MeOH = 8/2) |
| EX. 16 ⁽⁴⁾ | 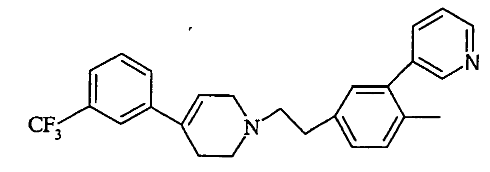 | TLC: R.f. 0,6 (AcEt) |

| | | |
|---|---|---|
| Notes: Les produits de départ sont décrits dans les Préparations ci-dessus ou bien ils peuvent être préparés de façon analogue à ceux-ci, notamment: (1) intermédiaire décrit dans la Préparation 8 (2 intermédiaire décrit dans la Préparation 9 (3) intermédiaires obtenus par réaction de l'acide 3-(2-hydroxyéthyl)-phényl-boronique avec des halo-hétérocycles appropriés (4) intermédiaire décrit dans la Préparation 10 | | |

Le solvant de réaction est de préférence la 4-méthyl-2-pentanone (au lieu de l'isopropanol décrit dans l'exemple 1)
TLC = chromatographie sur couche mince
AcEt = acétate d'éthyle
MeOH = méthanol

### EXEMPLE 17

### 1-[2-(3-(4-Pyridyl)-phényl)-éthyl]-4-(3-trifluoro-méthyl-phényl)-1,2,3,6-tétrahydro-pyridine 1-oxyde.

On prépare le dérivé N-oxyde du composé de l'Exemple 7 par réaction avec de l'acide *m-*chloro-perbenzoïque dans du chlorure de méthylène. Chromatographie sur couche mince (TLC): R.f. 0,15, éluant méthanol.

### EXEMPLE 18

### 1-[2-(3-(4-Pyridyl-1-oxyde)-phényl)-éthyl]-4-(3-trifluoro-méthyl-phényl)-1,2,3,6-tétrahydro-pyridine.

On mélange 0,24 g (0,001 mole) de 4-(3-trifluorométhyl-phényl)-1,2,3,6-tétrahydro-pyridine, 12 ml de méthanol, 0,26 ml d'acide acétique glacial, 0,12 g d'acétate de sodium. On refroidit jusqu'à 0-5°C et on y ajoute 8 ml de méthanol, 0,37 g de 3-(4-pyridyl-1-oxyde)-phényl-acétaldéhyde (tel qu'obtenu par la Préparation 11) et avec précaution, 0,2 g de cyanoborohydure de sodium. On agite pendant 0,5 heure à 0-5°C et ensuite une nuit à la température ambiante. On ajoute 3 ml d'acide chlorhydrique concentré, on agite pendant 15 minutes, on évapore le solvant sous pression réduite, on reprend le résidu avec un mélange acétate d'éthyle/NH₄OH dilué. On sèche la phase organique sur du sulfate de sodium, on filtre et on évapore le solvant. On purifie le résidu par chromatographie sur colonne de gel de silice en éluant par un mélange acétate d'éthyle/méthanol = 8/2. On obtient le composé du titre.
P.f. 142-145°C.

## Revendications

1. Composé de formule (I) : 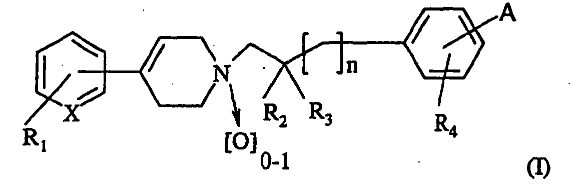 dans laquelle
X représente N ou CH ;
R₁ représente un atome d'hydrogène ou d'halogène ou un groupe CF₃;
R₂ et R₃ représentent indépendamment un atome d'hydrogène ou un groupe (C₁-C₄) alkyle;
R₄ représente un atome d'hydrogène ou un groupe (C₁-C₄) alkyle;
n est 0 ou 1 ;
A représente un hétérocycle azoté de formule (a)-(d) : 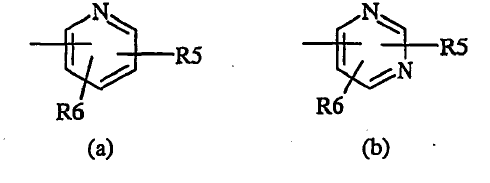 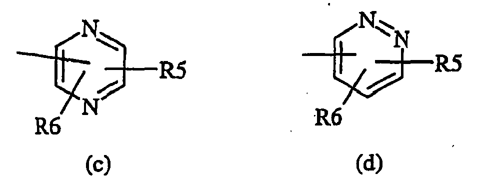
où R₅ et R₆ représentent chacun indépendamment un atome d'hydrogène,
un groupe (C₁-C₄) alkyle ou (C₁-C₄) alcoxy ;
ainsi que leurs N-oxydes et leurs sels ou solvates.

2. Composé selon la revendication 1, où n est zéro.

3. Composé selon la revendication 1, où R₂ et R₃ sont chacun l'hydrogène.

4. Composé selon le la revendication 1, où R₁ est un groupe CF₃.

5. Composé selon la revendication 1, où X est CH et R₁ est dans la position 3 du benzène.

6. Composé selon la revendication 1, où X est N et la pyridine ainsi formée est substituée dans les positions 2,6.

7. Composé selon la revendication 1, où le noyau phényle est substitué par l'hétérocycle azoté A dans la position 3.

8. Composé selon les revendications de 1 à 7, sous la forme de ses dérivés N-oxyde.

9. Composé choisi parmi:
1-[2-(4-(3-pyridyl)-phényl)-éthyl]-4-(3-trifluoro-méthyl-phényl)-1,2,3,6-tétrahydro-pyridine;
1-[2-(4-(2-pyridyl)-phényl)-éthyl]-4-(3-trifluoro-méthyl-phényl)-1,2,3,6-tétrahydro-pyridine;
1-[2-(4-(4-pyridyl)-phényl)-éthyl]-4-(3-trifluoro-méthyl-phényl)-1,2,3,6-tétrahydro-pyridine;
1-[2-(4-(6-méthyl-pyrid-3-yl)-phényl)éthyl]-4-(3-trifluoro-méthyl-phényl)-1,2,3,6-tétrahydro-pyridine;
1-[2-(3-(3-pyridyl)-phényl)-éthyl]-4-(3-trifluoro-méthyl-phényl)-1,2,3,6-tétrahydro-pyridine;
1-[2-(4-(5-pyrimidinyl)-phényl)-éthyl]-4-(3-trifluoro-méthyl-phényl)-1,2,3,6-tétrahydro-pyridine;
1-[2-(3-(4-pyridyl)-phényl)-éthyl]-4-(3-trifluoro-méthyl-phényl)-1,2,3,6-tétrahydro-pyridine;
1-[2-(3-(2-pyridyl)-phényl)-éthyl]-4-(3-trifluoro-méthyl-phényl)-1,2,3,6-tétrahydro-pyridine;
1-[2-(3-(2-pyridyl)-phényl)éthyl]-4-(6-trifluoro-méthyl-pyrid-2-yl)-1,2,3,6-tétrahydro-pyridine;
1-[2-(3-(2-pyridyl)-phényl)-éthyl]-4-(3-fluoro-phényl)-1,2,3,6-tétrahydropyridine;
1-[2-(3-(4-pyridyl)-phényl)-éthyl]-4-(6-chloro-pyrid-2-yl)-1,2,3,6-tétrahydro-pyridine;
1-[2-(3-(2-pyrazinyl)-phényl)-éthyl]-4-(3-trifluoro-méthyl-phényl)-1,2,3,6-tétrahydro-pyridine;
1-[2-(3-(6-méthyl-3-pyridyl)-phényl)éthyl]-4-(3-trifluoro-méthyl-phényl)-1,2,3,6-tétrahydro-pyridine;
1-[2-(3-(2-pyrimidinyl)-phényl)-éthyl]-4-(3-trifluoro-méthyl-phényl)-1,2,3,6-tétrahydro-pyridine;
1-[2-(3-(3-pyridazinyl)-phényl)-éthyl]-4-(3-trifluoro-méthyl-phényl)-1,2,3,6-tétrahydro-pyridine;
1-[2-(3-(3-pyridyl)-4-méthyl-phényl)-éthyl]-4-(3-trifluoro-méthyl-phényl)-1,2,3,6-tétrahydro-pyridine;
1-[2-(3-(4-pyridyl)-phényl)-éthyl]-4-(3-trifluoro-méthyl-phényl)-1,2,3,6-tétrahydro-pyridine 1-oxyde;
1-[2-(3-(4-pyridyl-1-oxyde)-phényl)-éthyl]-4-(3-trifluoro-méthyl-phényl)-1,2,3,6-tétrahydro-pyridine;
leurs sels et leurs solvates.

10. Procédé pour la préparation des composés de formule (I), **caractérisé en ce que** l'on fait réagir un composé de formule (II) 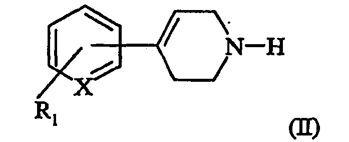 dans laquelle X et R₁ sont tels que définis ci-dessus, avec un composé de formule (III) : 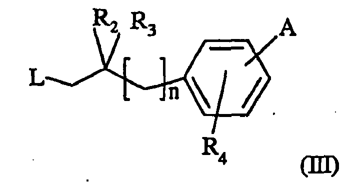 dans laquelle R₂, R₃, R₄, n et A sont tels que définis précédemment et L est un groupe partant, on isole le composé de formule (I) et on le transforme éventuellement en un de ses sels ou solvates ou dans ses dérivés N-oxydes.

11. Composition pharmaceutique contenant en tant que principe actif un composé de formule (I) selon les revendications de 1 à 9 ou un de ses sels ou solvates pharmaceutiquement acceptables.

12. Composition selon la revendication 11, **caractérisée en ce qu'**elle contient de 0,001 à 100 mg de principe actif.

13. Utilisation d'un composé de formule (I) selon les revendications de 1 à 9 pour la préparation de médicaments analgésiques et/ou destinés au traitement des maladies liées à des troubles immunitaires et inflammatoires.

14. Médicament contenant en tant que principe actif un composé de formule (I) selon les revendications de 1 à 9 ou un de ses sels ou solvates pharmaceutiquement acceptables.

15. Composé de formule (III) 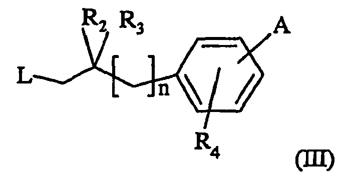
R₂ et R₃ représentent indépendamment un atome d'hydrogène ou un groupe (C₁-C₄) alkyle;
R₄ représente un atome d'hydrogène ou un groupe (C₁-C₄) alkyle;
n est 0 ou 1 ;
L représente un groupe partant;
A représente un hétérocycle azoté de formule (a)-(d) : 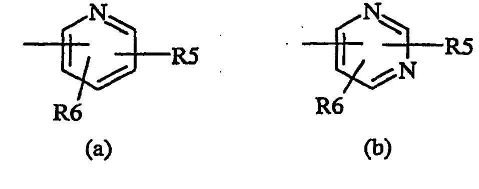 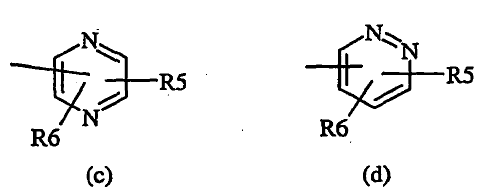
où R₅ et R₆ représentent chacun indépendamment un atome d'hydrogène, un groupe (C₁-C₄) alkyle ou (C₁-C₄) alcoxy ;
ainsi que leurs N-oxydes et leurs sels ou solvates.

## Claims

1. Compound of formula (I): 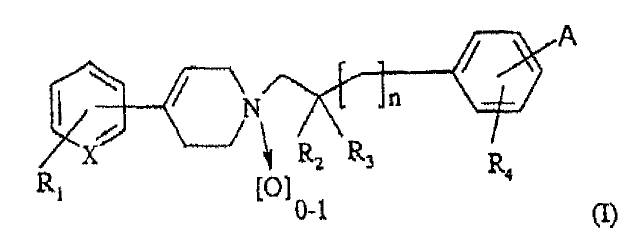 in which
X represents N or CH;
R₁ represents a hydrogen or halogen atom or a CF₃ group;
R₂ and R₃ independently represent a hydrogen atom or a (C₁-C₄) alkyl group;
R₄ represents a hydrogen atom or a (C₁-C₄) alkyl group;
n is 0 or 1;
A represents a nitrogenous heterocycle of formula (a)-(d): 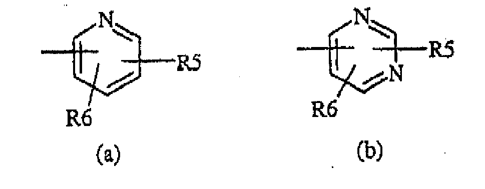 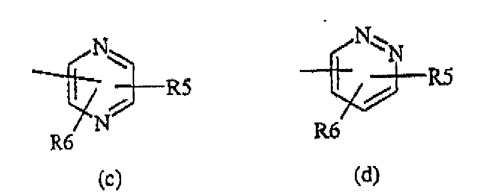 where R₅ and R₆ each independently represent a hydrogen atom or a (C₁-C₄) alkyl or (C₁-C₄) alkoxy group;
and its N-oxides and its salts or solvates.

2. Compound according to Claim 1, where n is zero.

3. Compound according to Claim 1, where R₂ and R₃ are each hydrogen.

4. Compound according to Claim 1, where R₁ is a CF₃ group.

5. Compound according to Claim 1, where X is CH and R₁ is in the 3-position of the benzene.

6. Compound according to Claim 1,
where X is N and the pyridine thus formed is substituted in the 2,6-positions.

7. Compound according to Claim 1, where the phenyl ring is substituted by the nitrogenous heterocycle A in the 3-position.

8. Compound according to Claims 1 to 7, in the form of its N-oxide derivatives.

9. Compound chosen from:
1- [2-(4-(3-Pyridyl) phenyl) ethyl]-4-(3-(trifluoromethyl)phenyl)-1,2,3,6-tetrahydropyridine;
1-[2-(4-(2-Pyridyl)phenyl)ethyl]-4-(3-(trifluoromethyl) phenyl)-1,2,3,6-tetrahydropyridine;
1-[2-(4-(4-Pyridyl)phenyl)ethyl]-4-(3-(trifluoromethyl) phenyl)-1,2,3,6-tetrahydropyridine;
1-[2-(4-(6-Methylpyrid-3-yl)phenyl)ethyl]-4-(3-(trifluoromethyl)phenyl)-1,2,3,6-tetrahydropyridine;
1-[2-(3-(3-Pyridyl)phenyl)ethyl]-4-(3-(trifluoromethyl)phenyl)-1,2,3,6-tetrahydropyridine;
1-[2-(4-(5-Pyrimidinyl)phenyl)ethyl]-4-(3-(trifluoromethyl)phenyl)-1,2,3,6-tetrahydropyridine;
1-[2-(3-(4-Pyridyl)phenyl)ethyl]-4-(3-(trifluoromethyl)phenyl)-1,2,3,6-tetrahydropyridine;
1-[2-(3-(2-Pyridyl)phenyl)ethyl]-4-(3-(trifluoromethyl)phenyl)-1,2,3,6-tetrahydropyridine;
1-[2-(3-(2-Pyridyl)phenyl)ethyl]-4-(6-(trifluoromethyl)pyrid-2-yl)-1,2,3,6-tetrahydropyridine;
1-[2-(3-(2-Pyridyl)phenyl)ethyl]-4-(3-fluorophenyl)-1,2,3,6-tetrahydropyridine;
1-[2-(3-(4-Pyridyl)phenyl)ethyl]-4-(6-chloropyrid-2-yl)-1,2,3,6-tetrahydropyridine;
1-[2-(3-(2-Pyrazinyl)phenyl)ethyl]-4-(3-(trifluoromethyl)phenyl)-1,2,3,6-tetrahydropyridine;
1-[2-(3-(6-Methyl-3-pyridyl)phenyl)ethyl]-4-(3-(trifluoromethyl)phenyl)-1,2,3,6-tetrahydropyridine;
1-[2-(3-(2-Pyrimidinyl)phenyl)ethyl]-4-(3-(trifluoromethyl)phenyl)-1,2,3,6-tetrahydropyridine;
1-[2-(3-(3-Pyridazinyl)phenyl)ethyl]-4-(3-(trifluoromethyl)phenyl)-1,2,3,6-tetrahydropyridine;
1-[2-(3-(3-Pyridyl)-4-methylphenyl)ethyl]-4-(3-(trifluoromethyl)phenyl)-1,2,3,6-tetrahydropyridine;
1-[2-(3-(4-Pyridyl)phenyl)ethyl]-4-(3-(trifluoromethyl)phenyl)-1,2,3,6-tetrahydropyridine 1-oxide;
1-[2-(3-(4-Pyridyl1-oxide)phenyl)ethyl]-4-(3-(trifluoromethyl)phenyl)-1,2,3,6-tetrahydropyridine; its salts and its solvates.

10. Process for the preparation of the compounds of formula (I), **characterized in that** a compound of formula (II) 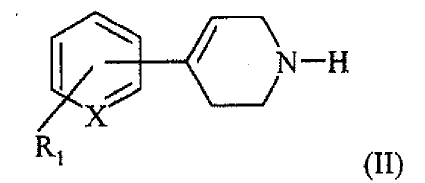 in which X and R₁ are as defined above, is reacted with a compound of formula (III): 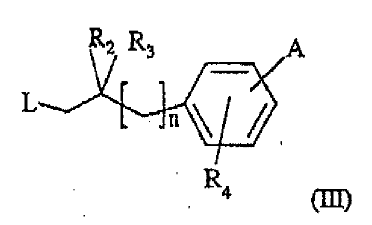 in which R₂, R₃, R₄, n and A are as defined above and L is a leaving group, the compound of formula (I) is isolated and is optionally converted to one of its salts or solvates or to its N-oxide derivatives.

11. Pharmaceutical composition, comprising, as active principle, a compound of formula (I) according to Claims 1 to 9 or one of its pharmaceutically acceptable salts or solvates.

12. Composition according to Claim 11, **characterized in that** it comprises from 0.001 to 100 mg of active principle.

13. Use of a compound of formula (I) according to Claims 1 to 9 for the preparation of analgesic medicaments and/or of medicaments intended for the treatment of diseases related to immune and inflammatory disorders.

14. Medicament, comprising, as active principle, a compound of formula (I) according to Claims 1 to 9 or one of its pharmaceutically acceptable salts or solvates.

15. Compound of formula (III) 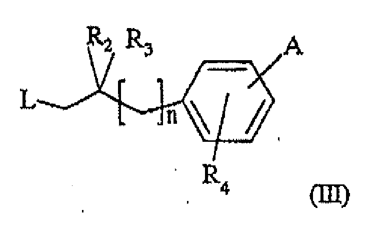 in which:
R₂ and R₃ independently represent a hydrogen atom or a (C₁-C₄)alkyl group;
R₄ represents a hydrogen atom or a (C₁-C₄) alkyl group;
n is 0 or 1;
L represents a leaving group;
A represents a nitrogenous heterocycle of formula (a) - (d) : 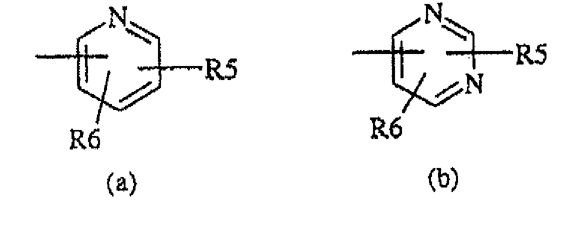 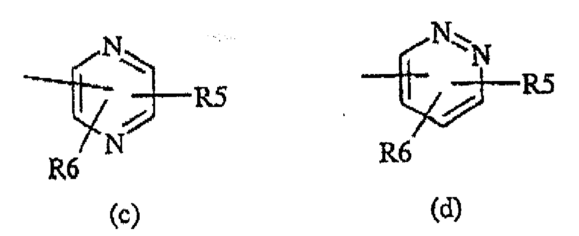
where R₅ and R₆ each independently represent a hydrogen atom or a (C₁-C₄ ) alkyl or (C₁-C₄) alkoxy group;
and its N-oxides and its salts or solvates.

## Patentansprüche

1. Verbindung der Formel (I): 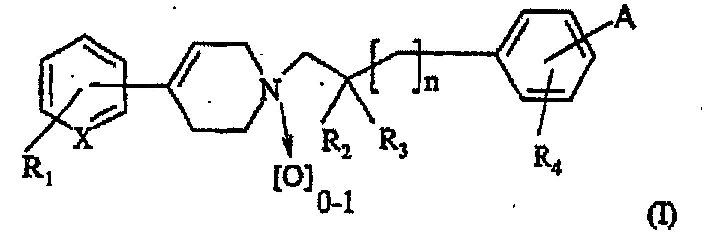 worin
X für N oder CH steht;
R₁ für ein Wasserstoff- oder Halogenatom oder eine CF₃-Gruppe steht;
R₂ und R₃ unabhängig voneinander für ein Wasser- stoffatom oder eine (C₁-C₄)-Alkylgruppe stehen;
R₄ für ein Wasserstoffatom oder eine (C₁-C₄)- Alkylgruppe steht;
n für 0 oder 1 steht;
A für einen stickstoffhaltigen Heterocyclus der Formel (a)-(d) steht: 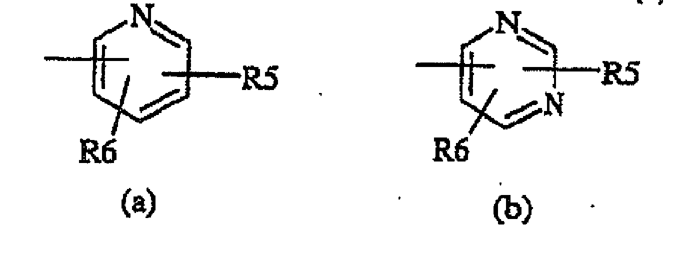 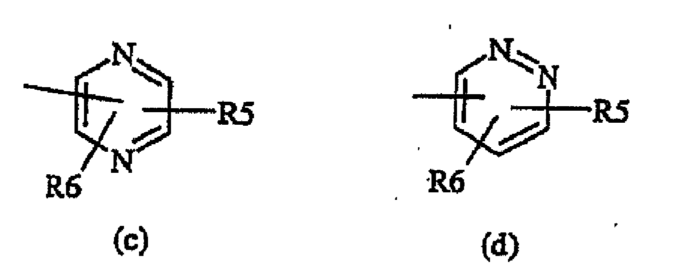
worin R₅ und R₆ unabhängig voneinander für ein Wasserstoffatom, eine (C₁-C₄)-Alkylgruppe oder eine (C₁-C₄)-Alkoxygruppe stehen;
sowie N-Oxide davon und Salze und Solvate davon.

2. Verbindung nach Anspruch 1, worin n für null steht.

3. Verbindung nach Anspruch 1, worin R₂ und R₃ jeweils für Wasserstoff stehen.

4. Verbindung nach Anspruch 1, worin R₁ für eine CF₃-Gruppe steht.

5. Verbindung nach Anspruch 1, worin X für CH steht und R₁ in der 3-Position des Benzols steht.

6. Verbindung nach Anspruch 1, worin X für N steht und das so gebildete Pyridin in den Positionen 2 und 6 substituiert ist.

7. Verbindung nach Anspruch 1, worin der Phenylring in der 3-Position durch den stickstoffhaltigen Heterocyclus A substituiert ist.

8. Verbindung nach den Ansprüchen 1 bis 7 in Form ihrer N-Oxid-Derivate.

9. Verbindung, ausgewählt unter:
1-[2-(4-(3-Pyridyl)phenyl)ethyl]-4-(3-(trifluormethyl)phenyl)-1,2,3,6-tetrahydropyridin;
1-[2-(4-(2-Pyridyl)phenyl)ethyl]-4-(3-(trifluormethyl)phenyl)-1,2,3,6-tetrahydropyridin;
1-[2-(4-(4-Pyridyl)phenyl)ethyl]-4-(3-(trifluormethyl)phenyl)-1,2,3,6-tetrahydropyridin;
1-[2-(4-(6-Methylpyrid-3-yl)phenyl)ethyl]-4-(3-(trifluormethyl)phenyl)-1,2,3,6-tetrahydropyridin;
1-[2-(3-(3-Pyridyl)phenyl)ethyl]-4-(3-(trifluormethyl)phenyl)-1,2,3,6-tetrahydropyridin;
1-[2-(4-(5-Pyrimidinyl)phenyl)ethyl]-4-(3-(trifluormethyl)phenyl)-1,2,3,6-tetrahydropyridin;
1-[2-(3-(4-Pyridyl)phenyl)ethyl]-4-(3-(trifluormethyl)phenyl)-1,2,3,6-tetrahydropyridin;
1-[2-(3-(2-Pyridyl)phenyl)ethyl]-4-(3-(trifluormethyl)phenyl)-1,2,3,6-tetrahydropyridin;
1-[2-(3-(2-Pyridyl)phenyl)ethyl]-4-(6-(trifluormethyl)pyrid-2-yl)-1,2,3,6-tetrahydropyridin;
1-[2-(3-(2-Pyridyl)phenyl)ethyl]-4-(3-fluorphenyl)-1,2,3,6-tetrahydropyridin;
1-[2-(3-(4-Pyridyl)phenyl)ethyl]-4-(6-chloropyrid-2-yl)-1,2,3,6-tetrahydropyridin;
1-[2-(3-(2-Pyrazinyl)phenyl)ethyl]-4-(3-(trifluormethyl)phenyl)-1,2,3,6-tetrahydropyridin;
1-[2-(3-(6-Methyl-3-pyridyl)phenyl)ethyl]-4-(3-(trifluormethyl)phenyl)-1,2,3,6-tetrahydropyridin;
1-[2-(3-(2-Pyrimidinyl)phenyl)ethyl]-4-(3-(trifluormethyl)phenyl)-1,2,3,6-tetrahydropyridin;
1-[2-(3-(3-Pyridazinyl)phenyl)ethyl]-4-(3-(trifluormethyl)phenyl)-1,2,3,6-tetrahydropyridin;
1-[2-(3-(3-Pyridyl)-4-methylphenyl)ethyl]-4-(3-(trifluormethyl)phenyl)-1,2,3,6-tetrahydropyridin;
1-[2-(3-(4-Pyridyl) phenyl) ethyl]-4-(3-(trifluormethyl)phenyl)-1,2,3,6-tetrahydropyridin-1-oxid;
1-[2-(3-(4-Pyridyl-1-oxid)phenyl)ethyl]-4-(3-(trifluormethyl)phenyl)-1,2,3,6-tetrahydropyridin;
Salzen davon und Solvaten davon.

10. Verfahren zur Herstellung von Verbindungen der Formel (I), **dadurch gekennzeichnet, daß** man eine Verbindung der Formel (II): 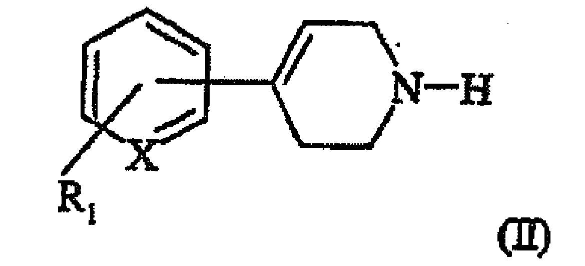 worin X und R₁ die oben angegebene Bedeutung besitzen, mit einer Verbindung der Formel (III): 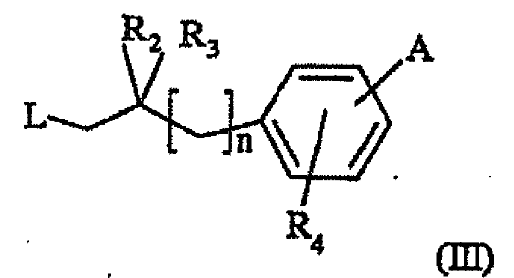 worin R₂, R₃ , R₄ , n und A die oben angegebene Bedeutung besitzen und L für eine Abgangsgruppe steht, die Verbindung der Formel (I) isoliert und gegebenenfalls in ein Salz oder Solvat davon oder in ein N-Oxid-Derivat davon umwandelt.

11. Pharmazeutische Zusammensetzung, enthaltend als Wirkstoff eine Verbindung der Formel (I) nach den Ansprüchen 1 bis 9 oder ein pharmazeutisch unbedenkliches Salz oder Solvat davon.

12. Zusammensetzung nach Anspruch 11, **dadurch gekennzeichnet, daß** sie 0,001 bis 100 mg Wikstoff enthält.

13. Verwendung einer Verbindung der Formel (I) nach den Ansprüchen 1 bis 9 zur Herstellung von analgetischen Arzneimitteln und/oder zur Behandlung von Erkrankungen, die mit Immunstörungen und entzündlichen Störungen verbunden sind, bestimmten Arzneimitteln.

14. Arzneimittel, enthaltend als Wirkstoff eine Verbindung der Formel (I) nach den Ansprüchen 1 bis 9 oder ein pharmazeutisch unbedenkliches Salz oder Solvat davon.

15. Verbindung der Formel (III) 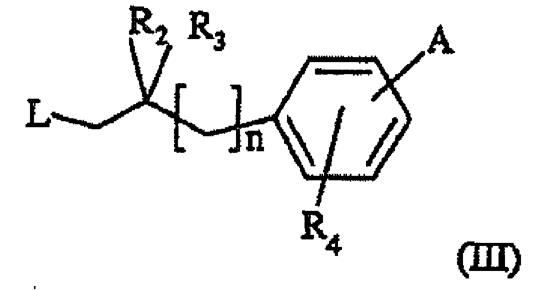 worin
R₂ und R₃ unabhängig voneinander für ein Wasserstoffatom oder eine (C₁-C₄)-Alkylgruppe stehen;
R₄ für ein Wasserstoffatom oder eine (C₁-C₄) - Alkylgruppe steht;
n für 0 oder 1 steht;
L für eine Abgangsgruppe steht;
A für einen stickstoffhaltigen Heterocyclus der Formel (a) - (d) steht: 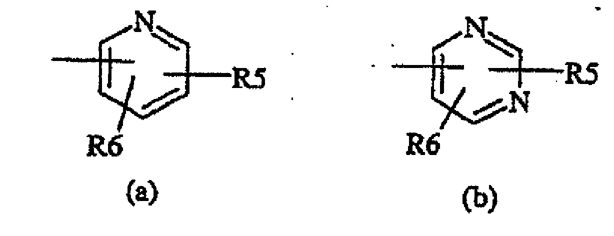 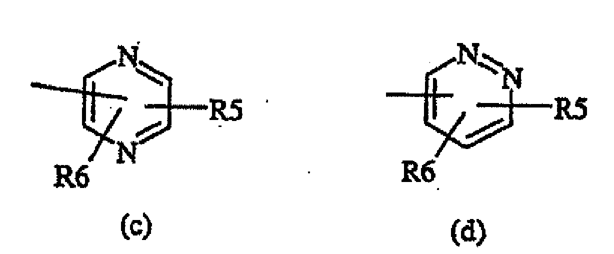
worin R₅ und R₆ unabhängig voneinander für ein Wasserstoffatom, eine (C₁-C₄)-Alkylgruppe oder eine (C₁-C₄) -Alkoxygruppe stehen;
sowie N-Oxide davon und Salze und Solvate davon.
